Europäisches Patentamt

European Patent Office

Office européen des brevets

(11) Publication number: **0 622 457 A1**

(12) **EUROPEAN PATENT APPLICATION**
**published in accordance with Art.**
**158(3) EPC**

(21) Application number: 93916174.1

(22) Date of filing: **16.07.93**

(86) International application number:
**PCT/JP93/00988**

(87) International publication number:
**WO 94/02603 (03.02.94 94/04)**

(51) Int. Cl.5: **C12N 15/10**

(30) Priority: **21.07.92 JP 215395/92**

(43) Date of publication of application:
**02.11.94 Bulletin 94/44**

(84) Designated Contracting States:
**CH DE FR GB LI SE**

(71) Applicant: **SUMITOMO ELECTRIC INDUSTRIES,**
**LTD.**
**5-33, Kitahama 4-chome,**
**Chuo-ku**
**Osaka-shi, Osaka 541 (JP)**

(72) Inventor: **NAKAMURA, Takeshi, Osaka Works**
**of Sumitomo**
**Electric Ind., Ltd.,**
**1-3, Shimaya 1-chome**
**Konohama-ku, Osaka-shi, Osaka 554 (JP)**
Inventor: **KISHIMOTO, Toshihiko, Osaka**
**Works of Sumitomo**
**Electric Ind., Ltd.,**
**1-3, Shimaya 1-chome**
**Konohana-ku, Osaka-shi, Osaka 554 (JP)**
Inventor: **ODA, Kinichiro, Dept. of Biological**
**Science and**

**Techn. of Science Univ. of Tokyo,**
**2641, Yamazaki**
**Noda, Chiba 378 (JP)**
Inventor: **NAKADA, Susumu, Dept. of**
**Biological Science and**
**Techn. of Science Univ. of Tokyo,**
**2641, Yamazaki**
**Noda-shi, Chiba 278 (JP)**
Inventor: **HARA, Eiji, Dept. of Biological**
**Science and**
**Techn. of Science Univ. of Tokyo,**
**2641, Yamazaki**
**Noda-shi, Chiba 278 (JP)**
Inventor: **TSURUI, Hiromichi, Dept. of Medical**
**Science of**
**Juntendo University,**
**2-1-1, Hongo**
**Bunkyo-ku, Tokyo 113 (JP)**

(74) Representative: **Kolb, Helga, Dr. Dipl.-Chem. et**
**al**
**Hoffmann, Eitle & Partner,**
**Patent-und Rechtsanwälte,**
**Arabellastrasse 4**
**D-81925 München (DE)**

(54) **METHODS OF SEPARATING CELL-SPECIFIC cDNA AND PREPARING LIBRARY THEREOF.**

(57) A method of separating a gene which is cell-specifically expressed by conducting subtraction between the genes expressed in at least two different kinds of cells.

## Fig.1

The present invention relates to a method for isolating and obtaining cDNA which is specific to a particular tissue or cell (hereinafter, the tissue and cell are referred to as "a cell" for simplicity purpose) by "subtraction". More specifically, the present invention relates to a method for isolating a gene which is specifically expressed in a particular cell by subjecting genes expressed in different two or more types of cell to substruction. The method for isolating a gene of the present invention is useful in gene manipulation, in particular, for preparing a cDNA library specific to a particular cell through acquisition of a cDNA corresponding to a gene specific to the cell and cloning the same.

As a method of analyzing a gene, it is a usual procedure to synthesize cDNAs from mRNAs which directly encode proteins and clone the same. Cloning and analyzing of cDNAs are useful in research on gene expression, development, and differentiation as well as in production of useful substances. Isolation of cell-specific cDNAs and preparation of a cell-specific cDNA library would provide a useful source for investigating a useful gene.

Subtracive hybridization for isolating, for example, a cDNA clone corresponding to mRNA which is expressed in mouse T cell but not in mouse B cell is well known in the art. However, it has been difficult according to the conventional method to efficiently obtain a gene that is cell-specifically expressed by subjecting genes expressed in different two or more types of cell to subtraction.

Brief description of the drawings

Figure 1 is a flow chart depicting a specific example of the first isolating method of the present invention.

Figure 2 is a flow chart depicting a specific example of the second isolating method of the present invention.

Figure 3 shows an advantage of a method utilizing a labeled primer (biotinyl oligo (dT) fragment) in a process of cDNA synthesis.

Figure 4 shows a disadvantage of a method utilizing an oligo (dT)-latex in a process of cDNA synthesis.

In the dwawings, the abbreviations have the following means.

(1): messenger RNA (mRNA)

(2): antisense strand cDNA

(3): sense strand cDNA

Bi: biotin

Fe/Av: a $Fe_2O_3$ particle coated with avidin

As a method for reparing a subtracted cDNA library, it has been recently proposed to use a oligo (dT)-latex made of a latex particle covalently linked to oligo (dT) primers (Nucleic Acids Research, Vol. 19, No. 25, 1991, p. 7097 - 7104).

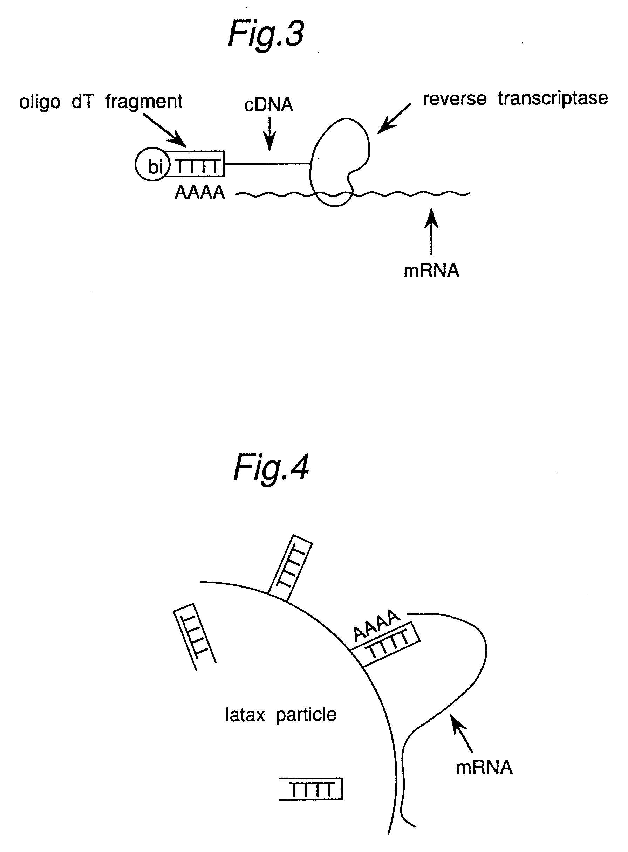

Since this method uses an oligo (dT)-latex as a means for isolating cDNAs from one of two types of cell, a certain region of mRNA, which is a template for synthesizing cDNA, may be non-specifically adsorbed to a latex particle, thereby inhibiting a synthesis of cDNA with a reverse transcriptase, as shown in Fig. 4, which causes a problem that such non-specific adsorption of mRNA onto the latex particle may interfere the preparation of a highly qualified library. Further, the method requires a large amount of expensive reverse transcriptase to prevent the template mRNAs from being adsorbed to a latex particle. The method also requires a lot of time for centrifugation, because the latex particle has a relatively small specific gravity.

Subject to be solved by the invention

An object of the present invention is to provide a method for isolating cell-specific cDNAs, which allows efficient and precise preparation of subtracted cDNA library specific to a particular cell.

Another object of the present invention is to provide a method for efficiently and precisely preparing cDNA library specific to a particular cell.

As the result of our research to overcome the above problems associated with the conventional method, the inventors have eventually found that the above-mentioned objects can be attained by combining a technique of synthesizing labeled cDNAs complementary to mRNAs expressed in a cell by the use of a labeled primer and a technique of specifically isolating the labeled cDNAs by the use of isolating particles which are capable of specifically binding to the label moiety in the labeled cDNAs, and by modifying the steps of subjecting genes that are expressed in different two or more types of cell to a subtraction. The present invention is based on such findings.

Means to solve the subject

According to the present invention, a method for isolating a gene that is expressed specifically in a particular cell by subjecting genes expressed in different two or more types of cell to subtraction, said method comprising the following steps, is provided:

(1) synthesizing labeled cDNAs that are complementary to mRNAs expressed in cell A by the use of a labeled primer, then binding said labeled cDNAs with isolating particles which are capable of specifically binding to the label moiety in the labeled cDNAs, and specifically isolating the resultant isolating particles/labeled cDNAs complexes;

(2) synthesizing labeled cDNAs that are complementary to mRNAs expressed in cell B by the use of a labeled primer, then binding said labeled cDNAs with isolating particles which are capable of specifically binding to the label moiety in the labeled cDNAs, and synthesizing DNAs that are complementary to the labeled cDNAs, and recovering the newly synthesized DNAs exclusively;

(3) mixing the isolating particles/labeled cDNAs complexes obtained in the step (1) with the synthesized DNAs recovered in the step (2) for hybridization; and

(4) recovering DNAs which are derived from the cell B and which did not hybridize in the step (3) (hereinafter, the above method will be referred to as "the first isolating method").

Another method for isolating genes that are expressed specifically in a particular cell by subjecting genes expressed in different two or more types of cell to subtraction, said method comprising the following steps, is also provided according to the present invention:

(1) synthesizing labeled cDNAs that are complementary to mRNAs expressed in cell A by the use of a labeled primer;

(2) synthesizing labeled cDNAs that are complementary to mRNAs expressed in cell B by the use of a labeled primer, synthesizing DNAs that are complementary to the labeled cDNAs, binding said labeled cDNAs with isolating particles which are capable of specifically binding to the label moiety in the labeled cDNAs to specifically isolate the labeled cDNAs, and recovering the newly synthesized DNAs exclusively;

(3) mixing the labeled cDNAs sythesized in the step (1) with the DNAs recovered in the step (2) for hybridization; and

(4) binding said labeled cDNAs synthesized in the step (1) to isolating particles which are capable of specifically binding to the label moiety in the labeled cDNAs to isolate the labeled cDNAs together with the DNAs which are derived from the cell B and which hybridized in the step (3), and recovering DNAs that are derived from the cell B and which did not hybridize in the step (3) (hereinafter, this method will be referred to as "the second isolating method").

Further, the present invention provides a method for preparing a cDNA library which is specific to a particular cell, the method being characterized in that, after the recovery of DNAs that are derived from the cell B and that did not hybridize in the step (3), the DNAs are further replicated to obtain cDNA clones corresponding to genes specific to the cell B in the step (4) of the above-mentioned isolating methods.

The present invention is explained in more detail below.

Primers comprising oligo (dT) complementary to a poly (A) portion of mRNA are generally used in synthesizing cDNAs from mRNAs in the presence of reverse transcriptase. In the present invention, labeled primers are used. Examples of labels used in labeled primers include biotin, antigens, substances capable of specifically binding to certain substances, and the like. That is, substances capable of forming specific bonds, such as a biotin-avidin bond and an antigen-antibody bond, can be used.

Examples of substances capable of specifically binding to a label moiety in a labeled primer include avidin, streptoavidin, antibody, and the like.

As isolating particles, there are used particles made of inorganic polymers, such as silica, ceramics, and the like; organic polymers, such as polystyrene, polypropylene, polyvinyl alcohol, and the like; metals, such as iron, copper, and the like; and metals coated with a substance capable of binding to a label moiety in a labeled cDNA, such as a complex particle made of a $Fe_2O_3$ particle coated by organic polymers, such as avidin.

Methods for isolating DNAs which have been bound, at the label moiety, with isolating particles include centrifugation, recovery by the use of magnets, filtration, and the like.

Conditions for hybridization, such as repeating times of hybridization, hybridization time, and hybridization temperature, can be chosen arbitrarily. Conditions of solutions, such as a solvent, a concentration of salts, and a ratio of labeled cDNAs from cell A to DNAs from cell B, can also be chosen arbitrarily.

In the present invention, a labeled primer having the label moiety which capable of specifically binding to an isolating particle is used to prepare cDNA containing the label at a terminal. By the use of such cDNA,

4

an isolating particle/labeled cDNA complex is formed in the present invention. The cDNA complex can be isolated from other substances in a vessel precisely and rapidly. The present invention does not have a disadvantage that mRNA, a template for cDNA synthesis, is non-specifically adsorbed to a latex particle, which inhibits the synthesis of cDNA as shown in Fig. 4, because, according to the present invention, cDNA is synthesized in a solution with the use of oligo (dT) fragment which is, for example, biotinated, as a primer as shown in Fig. 3.

An avidin-coated $Fe_2O_3$ particle (avidin/iron particle: Av/Fe), which can be used as an isolating particle, is bound to a biotinated oligo (dT) fragment primer, thereby forming a biotinated cDNA-avidin/iron particle complex. The complex hardly binds to the surface of a vessel containing a reaction mixture irreversibly. Therefore, the complex can be isolated from other substances in a reaction mixture rapidly and precisely with the use of a magnet. In such conditions, the supernatant of the reaction mixture can be easily recovered and solvents can be changed readily. The recovered supernatant rarely include the biotinated cDNA-avidin/iron particle complex, thus the complex is not lost when the supernatant is recovered. Therefore, subtracted cDNA library with high quality can be obtained from such cDNA.

Generally, it is effective to repeat each step of the isolating method in order to obtain subtracted cDNA library with high quality. However, it is not practical to repeat each step in the conventional method involving latex particles, because isolating step requires a lot of time to remove latex particles by centrifugation. The method of the present invention involving the isolating particles can be performed rapidly and precisely, and therefore, the isolating step can be repeated.

Further, when the labeled primer is used in cDNA synthesis as shown in Fig. 3, mRNA, a template for cDNA synthesis, is not non-specifically adsorbed to a latex particle (Fig. 4), and therefore, cDNA can be synthesized to a sufficient length. Thus, a cDNA library including cDNA clones with high quality can be obtained.

With reference to the accompanied drawings, the first and second isolating methods of the present invention are further explained below.

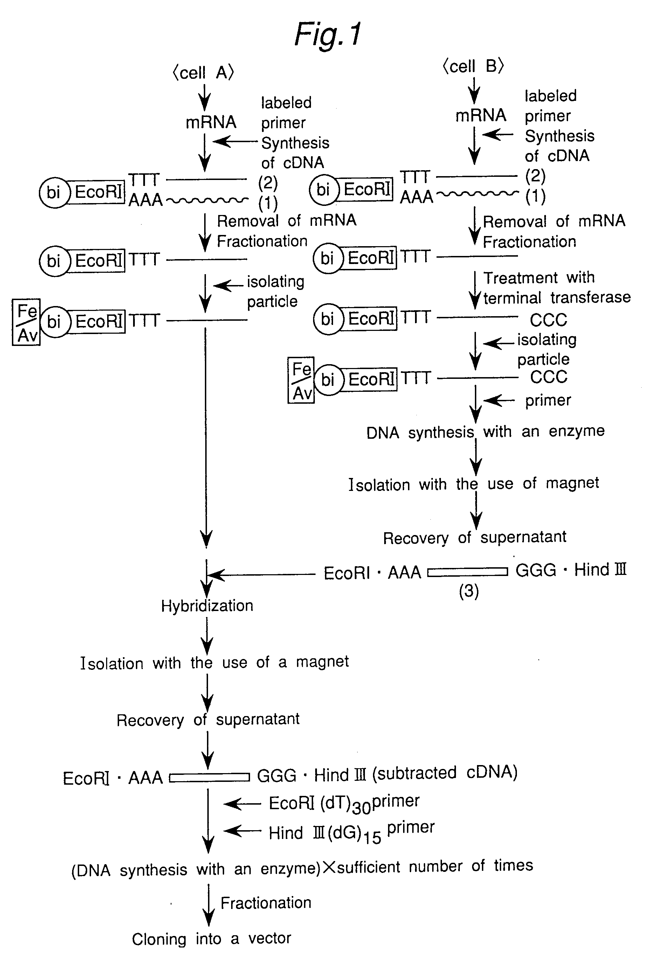

Fig.1 is a flow chart showing the first method for isolating cDNA and preparing cDNA library of the present invention.

Step 1

Preparation of labeled antisense strand cDNAs derived from mRNAs in cell A

(1) Mixing of the following components; mRNAs, labeled primers, reverse transcriptase, dNTPs, and the like.

(2) Synthesis of antisense strands, for example, at 42°C for 60 minutes.

(3) Removal of mRNAs, for example, by an alkali treatment.

(4) Removal of labeled primers that have not participated in the sythesis of antisense strands in the above procedure (2) by fractionation.

(5) Mixing of the product obtained by the procedure (4) with excess amount of isolating particles.

(6) Further blending of the mixture under rotation stirring, for example, for 30 minutes.

(7) Repeating washing as necessary (aggregation by the use of a magnet and exchange of buffer).

Step 2

Preparation of sense strand DNAs derived from mRNAs in cell B

Procedures from (1) to (4) are the same as those of Step 1.

(5) Mixing of the following components; antisense strand cDNAs, terminal transferase, dCTP, and the like.

(6) C terminal's filling by terminal transferase, for example, at 37°C for 5 minutes.

(7) Mixing of the product obtained by the procedure (6) with an excess amount of isolating particles.

(8) Futher blending of the mixture of the procedure (7) under rotation stirring.

(9) Repeating washing of the mixture as necessary.

(10) Addition of HindIII $(dG)_{15}$ primers.

(11) Repeating DNA synthesis with an enzyme twice.

(12) Aggregation with a magnet (or similar fractination procedure).

(13) Recovering the supernatant which includes sense strand DNAs.

## Step 3

A subtraction process between products of step 1 and step 2
(1) Mixing the product obtained by the procedure (4) in step 1 (labeled antisense strand cDNAs) and the product obtained by the procedure (13) in step 2 (sense strand DNAs).
(2) Hybridizing these products.

## Step 4

Recovery of DNAs derived from cell B that have not been hybridized in step 3
(1) Aggregation of the labeled DNAs with a magnet (or similar separation procedure).
(2) Recovery of the supernatant which includes subtracted sense strand DNAs.

## Step 5

Cloning and replication of cDNAs
(1) Adding EcoRI $(dT)_{30}$ primers and HindIII $(dG)_{15}$ primers for the following reaction procedure (2).
(2) Repeating DNA synthesis with an enzyme as necessary.
(3) Cloning DNAs in vectors.

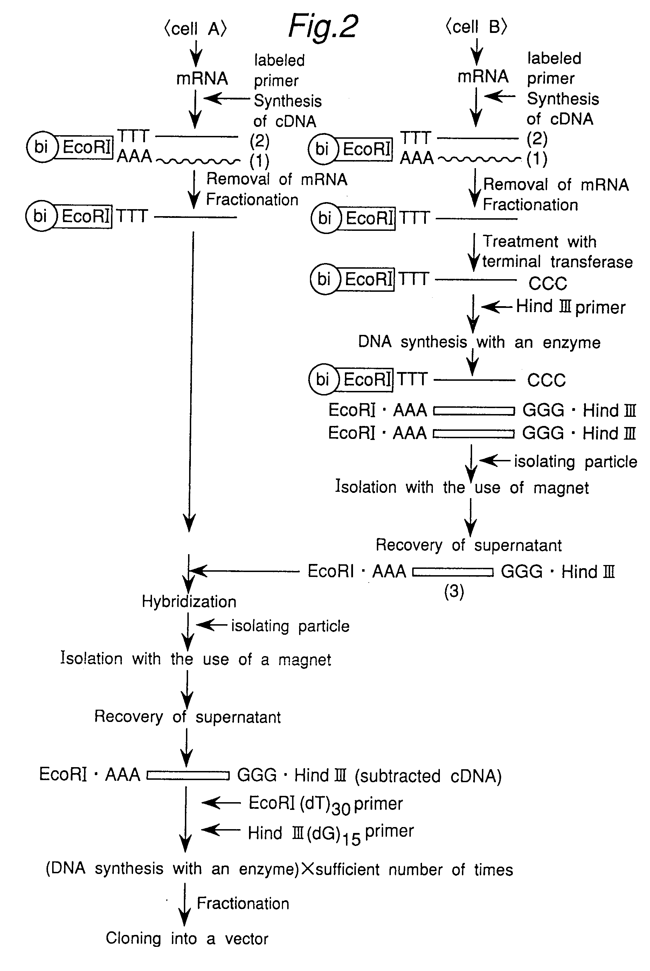

Fig. 2 shows the second method for isolating cDNAs and preparing a cDNA library of the present invention.

## Step 1

Preparation of labeled antisense strand cDNAs derived from mRNAs in cell A
(1) Mixing the following components; mRNA, a labeled primer, reverse transcriptase, dNTPs, and the like.
(2) Synthesis of antisense strands, for example, at 42°C for 60 minutes.
(3) Removal of mRNAs, for example, by an alkali treatment.
(4) Removal of labeled primers that have not participated in the sythesis of antisense strands in the procedure (2) by fractionation.

## Step 2

Preparation of sense strand DNAs derived from mRNAs in cell B
Procedures from (1) to (4) are the same as those of Step 1.
(5) Mixing the following components; antisense strand cDNAs, terminal transferase, dCTP, and the like.
(6) C terminal's addition by terminal transferase, for example, at 37° for 5 minutes.
(7) Addition of HindIII $(dG)_{15}$ primers.
(8) Repeating DNA synthesis with an enzyme twice.
(9) Mixing of the product obtained by (8) with an excess amount of isolating particles.
(10) Futher blending the mixture under rotation stirring, for example, for 30 minutes.
(11) Aggregation with a magnet (or similar fractionation procedure).
(12) Recovering the supernatant which includes sense strand DNAs.

## Step 3

A subtraction process between products of step 1 and step 2
(1) Mixing the product obtained by the procedure (4) in step 1 (labeled antisense strand cDNAs) and the product obtained by the procedure (12) in step 2 (sense strand DNAs).
(2) Hybridizing these products.

## Step 4

Recovery of DNAs derived from cell B that have not been hybridized in step 3
(1) Mixing the product obtained by the procedure (2) in step 3 and an excess amount of isolating particles.
(2) Further blending of the mixture of (1) under rotation stiring, for example, for 30 minutes.

(3) Aggregation with a magnet (or similar fractionation procedure).
(4) Recovering the supernatant which includes subtracted sense strand DNAs.

Step 5

Cloning and replication of cDNAs
(1) Addition of EcoRI (dT)$_{30}$ primers and HindIII (dG)$_{15w}$ primers for the following reaction (2).
(2) Repeating DNA synthesis with an enzyme as necessary.
(3) Cloning after incorporation into vector.

The above-mentioned methods of the present invention (methods for isolating genes and methods for preparing libraries) involve a single cell A and a single cell B, but a mixture of multiple types of cell A1, A2, ... can be used as cell A in the present application. In that case, mRNAs expressed in these cells are subtracted from mRNAs expressed in cell B, thereby a cDNA library specific to cell B can be prepared. Thus, the methods of the present invention can be applied to the isolation of genes specific to a particular cell by subtracting genes expressed in different two or more types of cell.

A specific linkage between a label moiety in a labeled primer and an isolating particle is sometimes unstable at high temperature. For example, linkage between biotin and avidin is very unstable at high temperature. Therefore, when biotin-labeled primer and avidin-coated isolating particle are used, and DNAs are hybridized with the avidin-coated particle/biotin-labeled cDNA complex in step 3 of the first isolating method at temperature of 60 to 75°C, usually at 65°C, the isolating particle tends to separate from the cDNA, which sometimes gives unsatisfactory results.

Such disadvantage as mentioned above can be overcomed by employing the second isolating method, wherein DNAs can be hybridized at 55 to 70°C, preferably 65°C, in the step 3, and binding to and separation from an isolating particle is conducted at 20 to 35°C, preferably 25 to 30°C, in the step 4, and binding an isolating particle to labeled cDNAs and separation thereof are conducted at 20 to 35°C, preferably 25 to 30°C, when the newly synthesized DNAs are recovered in step 2.

EXAMPLE

The present invention is further illustrated by the following examples. The exmaples are not to be construed as in any way limiting on the invention.

[Example 1]

The first isolating method and preparation of library were conducted by the use of young cells (Cell A) and sensecent cells (Cell B) of normal human fibroblast TIG cell according to the following procedures.

Step 1

(1) The following components were mixed; mRNA expressed in cell A (5 $\mu$g), biotinated oligo dT primers (1.5 $\mu$l), reverse transcriptase (24 units/$\mu$l, 3 $\mu$l), 6.6 mM dNTP (-dCTP) (5 $\mu$l), 0.66 mM dCTP (5 $\mu$l), [$\alpha$-$^{32}$P] dCTP (5 $\mu$l), buffer, and the like.
(2) The mixture of (1) was incubated at 42°C for 60 minutes.
(3) Five $\mu$l of 2N NaOH was added and the resulting mixture was incubated at 70°C for 20 minutes and at a room temperature for 15 minutes. Then, 10 $\mu$l of 1N HCl, 120 $\mu$l of 4 M ammonium acetate, and 600 $\mu$l of ethanol were added and the mixed solution was allowed to stand at -80°C for an hour. The solution was centrifuged for 20 minutes and the pellet was disolved in 100 $\mu$l of TE buffer.
(4) The primers were removed with the use of a Sephadex G-50 column (a molecular weight fractionating column).

At this stage, cDNAs were synthesized by the use of a labeled primer, which comprises oligo (dT) bearing biotin at a terminal, and an unlabeled primer, which comprises only oligo (dT), and then the amount and the length of the synthesized cDNAs were determined to verify the efficiency of the step 1 of the first isolating method of the present invention. The amount of the synthesized cDNAs were determined by the measurement of [$\alpha$-$^{32}$P] dCTPs, which had been incorporated into the cDNAs in a process of the cDNA synthesis, by a liquid scintillation counter.

The result of duplicate measurements revealed that the amount of the synthesized cDNAs by the use of the labeled primers was 1.09 times as large as that by the use of the unlabeled primers. Size distribution of the synthesized cDNAs was determined by an agarose electrophoresis. As a result, the size of the synthesized cDNAs by the use of the labeled primers was 1 to 1.5 kb, which was the same as that when the unlabeled primers were used. The amount and the size of synthesized cDNAs by the use of oligo (dT) latex are often smaller than those obtained by the use of oligo (dT) primers. The amount of reverse transcriptase required for cDNA synthesis by the use of a labeled primer is one tenth to one twentieth smaller than that when oligo (dT) latex is used.

(5) The following components were mixed; isolating particle (20 $\mu$l), which was prepared by coating $Fe_2O_3$ with avidin (Dynabeads, Dycell) and the product of (4)

(6) The mixture of (5) was further admixed under rotation stiring at room temperature for 30 minutes.

(7) The aggregation with a magnet was conducted as follow. The supernatant of the mixture was removed and the supernatant was measured by a liquid scintillation counter: ●new buffer [10 mM Tris/HCl, pH7.5, +1 mM EDTA, +NaCl (0.1 M or 1 M)] was added;

- the resulting mixture was suspended;
- aggregation with a magnet;
- a pellet was recovered from the aggregates and measured by a liquid scintillation counter.

The recovered pellet was "the labeled antisense strand cDNAs" to which isolating particles are bound.

At this stage, an experiment that illustrates the efficiency of aggregation and isolation of a cDNA complex with the use of a magnet was conducted. It is very important in the present invention that a cDNA complex consisting of an isolating particle/labeled cDNA can be readily and completely isolated. The experiment was done with a biotin-avidin system to determine whether this is true.

The aggregation and isolation of the cDNA complex was conpleted in a very short time (within a minute), while centrifugation of oligo (dT) latex requires 15 minutes Thus, the time requisite for the isolation is very shortened. Then, biotinated cDNAs were labeled with isotope and bound to isolating particles. After the biotinated cDNAs were suspended and isolated for four times, the amount of isolated labeled cDNAs were determined. As the result, 84% of the initial amount of labeled cDNAs were recovered in the presence of 0.1 M NaCl and 60% of the initial amount of labeled cDNAs were recovered in the presence of 1 M NaCl. Although the recovery of cDNAs was not complete because of the reason that a biotin-avidin linkage is unstable to some extent, the method involving the biotin-avidin system is practically useful.

## Step 2

Procedures from (1) to (4) are the same as those of Step 1, except that mRNAs from cell B were used.

(5) The following components are mixed;
- the product obtained by (4) (36 $\mu$l)
- 5 × reaction buffer (10 $\mu$l)
- 100 mM dCTP (1 $\mu$l)
- terminal transferase (13 units/$\mu$l, 3 $\mu$l).

(6) The mixture was incubated at 37°C for 5 minutes.

After the procedure of (6), $dG_{15}$ primers and $dT_{30}$ primers were added to the mixture and a DNA synthesis with an enzyme was repeated for 25 times to ascertain the fill-in of C terminal. The reaction mixture included the product obtained by the procedure (6) (1 $\mu$l), 1.25 mM dNTP (16 $\mu$l), 10 × reaction buffer (10 $\mu$l), 2.5 mM $MgCl_2$ (8 $\mu$l), $dG_{15}$ primers (1 $\mu$l), $dT_{30}$ primers (1 $\mu$l), an enzyme for synthesis (1 $\mu$l), and $H_2O$ (62 $\mu$l). The reaction was proceeded at 95°C for 1.5 minutes, at 45°C for 2 minutes, and at 72°C for 3 minutes. This process was repeated for 25 times.

The products before and after the reaction were agarose electrophoresed. Only the product after the reaction contained DNAs distributed to the region of 0.5 to 1.5 kb. This result reveals that the terminal transfer reaction was completed and the product of the procedure (6) was obtained.

## Step 3

(1) The product obtained by the step 1 (labeled antisense strand cDNAs) and the product obtained by the step 2 (sense strand DNAs) were mixed.

(2) Hybridization at 65°C.

## Step 4

(1) Aggregation with a magnet.

(2) Recovery of the supernatant which includes subtracted sense strand DNAs.

A cDNA library was prepared from the recovered supernatant by a standard method. The size distribution of cDNA clones contained in the recovered supernatant is an important index for showing a quality lebel of the library. cDNAs contained in the recovered supernatant were partially agarose electrophoresed before cloned into a vector in step 5. The size of the cDNAs were distributed from 0.5 kb to 2 kb, and major cDNAs are 1 kb to 1.5 kb.

As described above, genes that are expressed specifically in a senescent cell of normal human fibroblast TIG cell (cell B) were isolated by the use of a young cell of normal human fibroblast TIG cell (cell A) and the senescent cell by the isolating method of the present invention. Then, a library specific to the senescent cell was prepared by a method of the present invention. The resulting cDNA library was analyzed by a dot blot hybridization method to determine the amount of the amplified genes specific to the senescent cell.

The determination was done in duplicate in which subtractions were repeated twice and a product of each subtraction was analyzed. Amplification rate is shown in Table 1.

Amplification rate = [the amount of cDNAs from mRNAs in senescent cell]/[the amount of cDNAs from mRNAs in young cell]

|  | Subtraction 1 | Subtraction 2 |
|---|---|---|
| Determination 1 | 0.93 | 5.3 |
| Determination 2 | 0.96 | 3.5 |

The result shows that the cDNAs specific to a senescent cell were amplified by the duplicate subtractions. cDNA clones were taken from the subtracted cDNA library thus obtained to determine DNA sequences. A fiblonectin gene, procollagen gene, and the like, which are known to be contained in a senescent cell, were included in the determined clones. Therefore, the result shows that a subtracted cDNA library specific to a particular cell can be prepared by the method of the present invention.

The present invention enables the preparation of a subtracted cDNA library specific to a particular cell effectively and precisely.

The present invention does not have such a disadvantage that mRNA, a template for cDNA synthesis, is non-specifically absorbed to a latexparticle which inhibits a synthesis of cDNA, because labeled cDNAs can be synthesized with the use of a labeled primer from mRNAs specific to a certain cell. Therefore, the quality of the cDNA clones in a cDNA library is improved.

Further, cDNAs can be isolated from other components by a magnet, a centrifugation, or the like rapidly and completely, because an isolating particle/labeled cDNA complex is used in the present invention. Therefore, a subtracted cDNA library specific to a prticular cell can be prepared rapidly and precisely by the method of the present invention. The cDNA library thus obtained is very useful as a source of a novel gene.

**Claims**

1. A method for isolating genes that are expressed specifically in a particular cell by subjecting genes expressed in different two or more types of cell to subtraction, said method comprising the steps of

(1) synthesizing labeled cDNAs that are complementary to mRNAs expressed in cell A by the use of a labeled primer, then binding said labeled cDNAs with isolating particles which are capable of specifically binding to the label moiety in the labeled cDNAs, and specifically isolating the resultant isolating particles/labeled cDNAs complexes;

(2) synthesizing labeled cDNAs that are complementary to mRNAs expressed in cell B by the use of a labeled primer, then binding said labeled cDNAs with isolating particles which are capable of specifically binding to the label moiety in the labeled cDNAs, and synthesizing DNAs that are complementary to the labeled cDNAs, and recovering the newly synthesized DNAs exclusively;

(3) mixing the isolating particles/labeled cDNAs complexes obtained in the step (1) with the synthesized DNAs recovered in the step (2) for hybridization; and

(4) recovering DNAs which are derived from the cell B and which did not hybridize in the step (3).

2. A method for isolating genes that are expressed specifically in a particular cell by subjecting genes expressed in different two or more types of cells to subtraction, said method comprising the steps of

(1) synthesizing labeled cDNAs that are complementary to mRNAs expressed in cell A by the use of a labeled primer;

(2) synthesizing labeled cDNAs that are complementary to mRNAs expressed in cell B by the use of a labeled primer, synthesizing DNAs that are complementary to the labeled cDNAs, binding said labeled cDNAs with isolating particles which are capable of specifically binding to the label moiety in the labeled cDNAs to specifically isolate the labeled cDNAs, and recovering the newly synthesized DNAs exclusively;

(3) mixing the labeled cDNAs synthesized in the step (1) with the DNAs recovered in the step (2) for hybridization; and

(4) binding said labeled cDNAs synthesized in the step (1) to isolating particles which are capable of specifically binding to the label moiety in the labeled cDNAs to isolate the labeled cDNAs together with the DNAs which are derived from the cell B and which hybridized in the step (3), and recovering DNAs that are derived from the cell B and which did not hybridize in the step (3).

3. The method for isolating genes according to claim 1 or 2, wherein said label moiety in the labeled primer is biotin, and said isolating particle is a complex particle which is made of a particle capable of being recovered by a magnet and avidin which coats said particle.

4. The method for isolating genes according to claim 3, wherein said complex particle is made of a $Fe_2O_3$ particle coated with avidin.

5. The method for isolating genes according to claim 2, wherein the hybridization process in the step (3) is conducted at 55 to 70°C and the binding process with the isolating particles and the recovering process from the particles in the step (4) is conducted at 20 to 35°C.

6. The method for isolating genes according to claim 2, wherein the binding process and the isolating process between isolating particles and labeled cDNAs is conducted at 20 to 35°C when the newly synthesized DNAs are recovered in the step (2).

7. A method for preparing a cDNA library which is specific to a particular cell characterized in that after recovery of DNAs in the step (4) of claim 1 or 2, which are derived from the cell B and which have not been hybridized in the step (3), the DNAs are further replicated to obtain cDNA clones corresponding to specific genes for the cell B.

## Fig.1

*Fig.2*

⟨cell A⟩

mRNA

labeled primer
— Synthesis of cDNA

(bi)EcoRI TTT ——————— (2)
AAA ∿∿∿∿∿ (1)

Removal of mRNA
Fractionation

(bi)EcoRI TTT ———————

⟨cell B⟩

mRNA

labeled primer
— Synthesis of cDNA

(bi)EcoRI TTT ——————— (2)
AAA ∿∿∿∿∿ (1)

Removal of mRNA
Fractionation

(bi)EcoRI TTT ———————

Treatment with terminal transferase

(bi)EcoRI TTT ——————— CCC

← Hind III primer

DNA synthesis with an enzyme

(bi)EcoRI TTT ——————— CCC
EcoRI · AAA ⊏▭⊐ GGG · Hind III
EcoRI · AAA ⊏▭⊐ GGG · Hind III

← isolating particle

Isolation with the use of magnet

Recovery of supernatant

——— EcoRI · AAA ⊏▭⊐ GGG · Hind III
(3)

Hybridization

← isolating particle

Isolation with the use of a magnet

Recovery of supernatant

EcoRI · AAA ⊏▭⊐ GGG · Hind III (subtracted cDNA)

← EcoRI (dT)$_{30}$ primer
← Hind III (dG)$_{15}$ primer

(DNA synthesis with an enzyme)✕sufficient number of times

Fractionation

Cloning into a vector

# Fig.3

oligo dT fragment      cDNA      reverse transcriptase

bi TTTT

AAAA

mRNA

# Fig.4

TTTT

TTTT

AAAA
TTTT

latax particle

mRNA

TTTT

| | International application No. |
|---|---|
| | PCT/JP93/00988 |

**A. CLASSIFICATION OF SUBJECT MATTER**

Int. Cl$^5$  C12N15/10

According to International Patent Classification (IPC) or to both national classification and IPC

**B. FIELDS SEARCHED**

Minimum documentation searched (classification system followed by classification symbols)

Int. Cl$^5$  C12N15/00

Documentation searched other than minimum documentation to the extent that such documents are included in the fields searched

WPI, BIOSIS PREVIEWS : subtract, hybridize, mRNA, cDNA

Electronic data base consulted during the international search (name of data base and, where practicable, search terms used)



**C. DOCUMENTS CONSIDERED TO BE RELEVANT**

| Category* | Citation of document, with indication, where appropriate, of the relevant passages | Relevant to claim No. |
|---|---|---|
| A | Biological Abstracts, Vol. 93, No. 087977 Hara E et al. : "Subtractive cDNA Cloning Using Oligo-DT-30-Latex and PCR Isolation and cDNA Clones Specific to Undifferentiated human Embryonal Carcinoma Cells" & Nucleic Acids Res 19(25), 1991, 7097-7104 | 1-7 |
| A | Biological Abstracts, Vol. 93, No. 012829, Kho C-J; Zarbl H : "A Rapid and Efficient Method for The Genevation of a Subtracted cDNA Library" & Technigue (Phila) 3(2)-1991, 58-63 | 1-7 |
| A | Biological Abstracts, Vol. 90, No. 042562, Krady JK et al. : "Use of Avidin Biotin Subtractive hybridization to characterize Messenger RNA Common to Neutons Destroyed by the Selective Neurotoxicant Trimethyltin" & Mol Brain Res 7(4), 1990, 287-298 | 1-7 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| August 13, 1993 (13. 08. 93) | September 7, 1993 (07. 09. 93) |

| Name and mailing address of the ISA/ | Authorized officer |
|---|---|
| Japanese Patent Office | |
| Facsimile No. | Telephone No. |

Form PCT/ISA/210 (second sheet) (July 1992)